(19) Europäisches Patentamt
European Patent Office
Office européen des brevets

(11) Veröffentlichungsnummer : **0 344 660 B1**

(12) # EUROPÄISCHE PATENTSCHRIFT

(45) Veröffentlichungstag der Patentschrift :
**22.07.92 Patentblatt 92/30**

(51) Int. Cl.$^5$ : **C07D 333/22,** C07D 333/28, C07D 409/14, A01N 43/10

(21) Anmeldenummer : **89109592.9**

(22) Anmeldetag : **27.05.89**

(54) **Thiophenverbindungen.**

(30) Priorität : **01.06.88 DE 3818670**

(43) Veröffentlichungstag der Anmeldung :
**06.12.89 Patentblatt 89/49**

(45) Bekanntmachung des Hinweises auf die Patenterteilung :
**22.07.92 Patentblatt 92/30**

(84) Benannte Vertragsstaaten :
**CH DE ES FR GB IT LI NL**

(56) Entgegenhaltungen :
**FR-A- 2 255 896**
**US-A- 3 050 442**
**CHEMICAL ABSTRACTS, Band 78, Nr. 23, 11. Juni 1973, Seite 359, Zusammenfassung 147712c, Columbus, Ohio, US; K.I. VAKHREEVA etal.: "Synthesis of 2,2'-bithiophene azomethine bases with potential biological activity", KHIM.-FARM. ZH. 1973, 7(3), 24-8**
**CHEMICAL ABSTRACTS, Band 76, Nr. 25, 19. Juni 1972, Seite 439, Zusammenfassung Nr. 153461v, Columbus, Ohio, US; K.I.VAKHREEVA et al.: "Synthesis of azomethine bases of the 2,2'-bithiophene series", KHIM.-FARM. ZH. 1972, 6(1), 24-5**
**ZEITSCHRIFT FÜR NATURFORSCHUNG, Band 41b, Nr. 6, Juni 1986, Teil B, Anorganische Chemie, Organische Chemie, Seiten 751-761,Verlag der Zeitschrift für Naturforschung, Tübingen, DE; G. KOSSMEHL et al.: "Flüssig-kristalline Verbindungen aus derThiophenreihe, Teil 3 Azomethine und Vinylene"**
**CHEMICAL ABSTRACTS, Band 88, 1978, Seite 490, Zusammenfassung Nr. 5713m, Columbus, Ohio, US; N.A. KABO et al.: "Manifestationof absorption K-bands in UV absorption spectra of azomethine bases", KHIM. GETEROTSIKL. SOEDIN. 1977, (8), 1043-6**

(56) Entgegenhaltungen :
**CHEMICAL ABSTRACTS, Band 110, Nr. 10, 6. März 1989, Seite 688, Zusammenfassung Nr. 85477y, Columbus, Ohio, US; & JP-A-63167 370 (FUJI ELECTRIC CO., LTD) 11-07-1988**
**CHEMICAL ABSTRACTS, Band 110, Nr. 8, 20. Februar 1989, Seite 624, Zusammenfassung Nr. 66881j, Columbus, Ohio, US; &JP-A-63 157 160 (FUJI ELECTRIC CO., LTD) 30-06-1988**
**CHEMICAL ABSTRACTS, Band 110, Nr. 8, 20. Februar 1989, Seite 622, Zusammenfassung Nr. 66857f, Columbus, Ohio, US; &JP-A-63 183 448 (FUJI ELECTRIC CO., LTD) 28-07-1988**

(73) Patentinhaber : **BASF Aktiengesellschaft**
**Carl-Bosch-Strasse 38**
**W-6700 Ludwigshafen (DE)**

(72) Erfinder : **Kober, Reiner, Dr.**
**Im Schlittweg 20**
**W-6701 Fussgoenheim (DE)**
Erfinder : **Leyendecker, Joachim. Dr**
**Stahlbuehlring 79**
**W-6802 Ladenburg (DE)**
Erfinder : **Seele, Rainer, Dr.**
**Leiblstrasse 3**
**W-6701 Fussgoenheim (DE)**
Erfinder : **Karbach, Stefan, Dr.**
**Grundwiesenweg 44**
**W-6730 Neustadt (DE)**
Erfinder : **Meyer, Norbert, Dr.**
**Dossenheimer Weg 22**
**W-6802 Ladenburg (DE)**
Erfinder : **Westphalen, Karl-Otto, Dr.**
**Mausbergweg 58**
**W-6720 Speyer (DE)**
Erfinder : **Wuerzer, Bruno, Dr.**
**Ruedigerstrasse 13**
**W-6701 Otterstadt (DE)**
Erfinder : **Wagenblast, Gerhard, Dr.**
**Hanns-Fay-Strasse 3**
**W-6710 Frankenthal (DE)**

**EP 0 344 660 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Thiophenverbindungen der allgemeinen Formel I

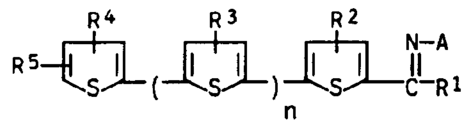

(I),

in der die Substituenten und der Index folgende Bedeutung haben:

n 0 oder 1;

$R^1$ Wasserstoff, ein Halogenatom, eine $C_1$-$C_8$-Alkylgruppe, eine $C_1$-$C_6$-Alkoxygruppe, eine $C_1$-$C_8$-Halogenalkylgruppe oder eine $C_1$-$C_6$-Halogenalkoxygruppe;

$R^2$, $R^3$, $R^4$ und $R^5$ Cyano, Nitro oder die für $R^1$ genannten Gruppen;

A Wasserstoff, eine $C_1$-$C_8$-Alkylgruppe, eine $C_1$-$C_6$-Halogenalkylgruppe, eine $C_1$-$C_8$-Alkoxygruppe, eine $C_1$-$C_6$-Halogenalkoxygruppe, eine Arylgruppe oder eine Heteroarylgruppe, wobei die aromatischen Gruppen ein bis fünf Halogenatome und/oder ein bis drei der folgenden Reste tragen können: $C_1$-$C_8$-Alkyl, $C_1$-$C_6$-Halogenalkyl, $C_1$-$C_8$-Alkoxy, $C_1$-$C_6$-Halogenalkoxy, Hydroxycarbonylamino und/oder $C_1$-$C_4$-Alkoxycarbonylamino; ein Rest $OR^6$ worin

$R^6$ Wasserstoff, $C_1$-$C_8$-Alkyl, $C_1$-$C_4$-Alkyl, welches ein- bis fünffach durch Halogen und/oder einfach durch einen der folgenden Reste substituiert ist: Cyano, $C_3$-$C_7$-Cycloalkyl, Hydroxy, $C_1$-$C_4$-Alkoxy, $C_1$-$C_4$-Alkylcarbonyl-$C_1$-$C_4$-alkoxy, $C_3$-$C_7$-Cycloalkylcarbonyl-$C_1$-$C_4$-alkoxy, $C_1$-$C_8$-Alkylcarbonyl, $C_1$-$C_8$-Halogenalkylcarbonyl, $C_3$-$C_8$-Cycloalkylcarbonyl, $C_1$-$C_8$-Alkoxycarbonyl, Mercapto, $C_1$-$C_4$-Alkylthio, Amino, $C_1$-$C_6$-Alkylamino, Di-$C_1$-$C_6$-Alkylamino, wobei diese Alkylgruppen auch gemeinsam mit dem Stickstoffatom einen aliphatischen Ring bilden können, Amino-$C_1$-$C_4$-alkoxy, $C_1$-$C_4$-Alkylamino-$C_1$-$C_4$-alkoxy, Di-$C_1$-$C_4$-Alkylamino-$C_1$-$C_4$-alkoxy, wobei die Alkylgruppen auch gemeinsam mit dem Stickstoffatom einen aliphatischen Ring bilden können oder fünf- oder sechsgliedriges Heteroaryl, enthaltend ein bis drei der Heteroatome Stickstoff, Sauerstoff und/oder Schwefel, wobei dieser Cyclus seinerseits ein- bis fünffach durch Halogen und/oder ein- bis dreifach durch folgende Reste substituiert sein kann: $C_1$-$C_4$-Alkyl, $C_1$-$C_4$-Alkoxy, $C_1$-$C_4$-Halogenalkyl und/oder $C_1$-$C_4$-Halogenalkoxy; $C_3$-$C_9$-Cycloalkyl; $C_2$-$C_8$-Alkenyl, welches ein- bis fünffach durch Halogen und/oder einfach durch $C_1$-$C_4$-Alkoxy substituiert sein kann; $C_3$-$C_7$-Alkinyl, welches ein- bis fünffach durch Halogen und/oder einfach durch $C_1$-$C_4$-Alkoxy substituiert sein kann;

bedeutet;

oder ein Rest $NR^7R^8$, worin

$R^7$, $R^8$ Wasserstoff, $C_1$-$C_8$-Alkyl, $C_1$-$C_8$-Alkoxy, $C_1$-$C_6$-Halogenalkyl, $C_1$-$C_6$-Halogenalkoxy, Aryl oder Hetaryl, die ein- bis dreifach duch Halogen, $C_1$-$C_8$-Alkyl, $C_1$-$C_8$-Alkoxy, $C_1$-$C_6$-Halogenalkyl, $C_1$-$C_6$-Halogenalkoxy oder Alkoxycarbonylamino substituiert sein können, $C_1$-$C_{12}$-Alkylcarbonyl, $C_1$-$C_{12}$-Halogenalkylcarbonyl, Aroyl oder Hetaroyl, welche ein- bis bis dreifach durch Halogen, $C_1$-$C_5$-Alkoxy, $C_1$-$C_4$-Halogenalkyl oder $C_1$-$C_6$-Alkyl substituiert sein können bedeutet,

sowie deren umweltverträglichen Salze;

mit der Maßgabe, daß, wenn n für 0 steht,

a) A keine ggf. substituierte Aryl- oder Heteroarylgruppe bedeutet, wenn $R^4$ Wasserstoff und gleichzeitig $R^5$ Wasserstoff, Methyl oder Nitro bedeuten und

b) $R^7$ oder $R^8$ nicht für Phenyl oder substituiertes Phenyl steht und $R^7$ und $R^8$ nicht gleichzeitig Phenyl bedeuten, wenn $R^1$, $R^2$, $R^4$ und $R^5$ Wasserstoff bedeuten.

Des weiteren betrifft die Erfindung Verfahren zur Herstellung dieser Verbindungen sowie ihre Verwendung in herbiziden Mitteln.

In Zeitschrift für Naturforschung, Bd. 41b, Nr. 6, 1986, Teil B, Seiten 751 bis 756, Verlag der Zeitschrift für Naturforschung, Tübingen sowie in Chem. Abstracts, Bd. 78, 1973, Seite 359, Zusammenfassung Nr. 147712c und Chem. Abstracts, Bd. 88, 1978, Seite 490, Zusammenfassung Nr. 5713m werden Azomethine, die das 2,2'-Bithienyl-System enthalten und am Stickstoff durch eine ggf. substituierte Aryl- oder Heteroarylgruppe substituiert sind, offenbart. Ferner sind Chem. Abstracts, Bd. 110, Nr. 10, 1989, Seite 688, Zusammenfassung Nr. 85477y Hydrazone vom Bithienyl-Typ zuu entnehmen. Keine der voranstehenden Literaturstellen legt eine herbizide Verwendung der offenbarten Bithienylderivate nahe.

In der US-PS 3 050 442 werden Bi- und Terthienyl-(Bis- und Terthiophen)- Verbindungen als Mittel zur Bekämpfung von Nematoden beschrieben. An einigen dieser Bi- und Terthienyl-Verbindungen wurde eine herbizide Wirkung beobachtet, die aber unbefriedigend war.

Der Erfindung lag daher die Aufgabe zugrunde neue Thiophenverbindungen mit verbesserten herbiziden Eigenschaften zu finden.

Dementsprechend wurden die eingangs definierten Thiophenverbindungen I gefunden. Außerdem wurde ein Verfahren zur Herstellung der Verbindungen I sowie deren Verwendung zur Bekämpfung unerwünschten Pflanzenwachstums gefunden.

Man erhält die neuen Verbindungen I analog zu literaturbekannten Methoden beispielsweise dadurch, daß man eine entsprechende Thiophenverbindung der Formel II

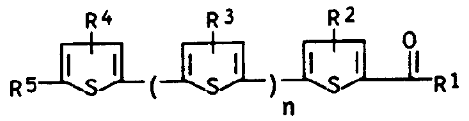

II,

in an sich bekannter Weise mit einer entsprechenden Hydroxylaminoverbindung (oder der -ammoniumverbindung und einer Base) bzw. einem entsprechend substituierten Hydrazin bzw. einem entsprechend substituierten primären Amin umsetzt.

Im Hinblick auf ihre biologische Wirksamkeit haben die Substituenten in Formel I bevorzugt die folgende Bedeutung:

$R^1$ Wasserstoff, ein Halogenatom wie Fluor, Chlor, Brom und Iod, vorzugsweise Chlor, eine Alkylgruppe wie Methyl, Ethyl, Propyl, 1-Methylethyl, Butyl, 1-Methylpropyl, 2-Methylpropyl, 1,1-Dimethylethyl,
eine Alkoxygruppe wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy,
eine Alkylthiogruppe wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio, eine Halogenalkylgruppe wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, oder eine entsprechende Halogenalkoxygruppe,

$R^2$, $R^3$, $R^4$ und $R^5$ Cyano, Nitro oder die bei $R^1$ im allgemeinen und im besonderen genannten Reste,

A Wasserstoff, eine Alkyl-, Halogenalkyl-, Alkoxy- oder Halogenalkoxygruppe wie im allgemeinen und im besonderen bei $R^1$ genannt, eine Aryl- oder Heteroarylgruppe wie Phenyl, Naphthyl, Pyrrolyl, Pyrazolyl, Imidazolyl, Isoxazolyl, Oxazolyl, Isothiazolyl, Thiazolyl, Furanyl und Thienyl, Pyridyl, Pyridazinyl, Pyrimidinyl, Pyrazinyl, Indolyl, Isoindolyl, Chinolinyl, Isochinolinyl und Pruinyl, insbesondere Phenyl, Fury, Pyridyl und Thienyl, wobei diese aromatischen Gruppen ein bis fünf der unter $R^1$ genannten Halogenatome, insbesondere Fluor, Chlor und/oder Brom und/oder ein bis drei der folgenden Reste tragen können: Alkyl, Halogenalkyl, Alkoxy und Halogenalkoxy wie bei $R^1$ genannt, insbesondere Methyl, 1-Methylethyl, Difluormethyl, Trifluormethyl, Chlormethyl, Methoxy, Ethoxy, Isopropyloxy, Difluormethoxy und Trifluormethoxy sowie Alkoxycarbonylamino wie Methoxycarbonylamino, Ethoxycarbonylamino, Propyloxycarbonylamino, 1-Methylethoxycarbonylamino und 1,1-Dimethylethoxycarbonylamino,

ein Rest $OR^6$, worin

$R^6$ Wasserstoff, Alkyl wie bei $R^1$ genannt, insbesondere Methyl, Ethyl, Propyl und Isopropyl, Alkenyl wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butnyl, 2-Methyl-3-butenyl, 3-Methyl-3-butenyl, 1,1-Dimethyl-2-propenyl, 1,-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Mthyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, insbesondere Propen-2-yl und Buten-2-yl, Halogenalkenyl wie insbesondere 3-Chlorpropen-2-yl, 3,3-Dichlorpropen-2-yl und 4,4-Dichlorbuten-2-yl, primäres, sekundäres oder tertiäres aliphatisches cyclisches oder acyclisches Aminoalkyl oder Aminoalkoxyalkyl wie insbesondere 2-Methylaminoethyl, 2,2-Dimethylaminoethyl, 3,3-Dimethylaminopropyl, N-Methylaziridylmethyl, 2-Aminoethyl, 2-(2-Aminoethoxy)ethyl, 2-(Methylamino)-propyl, Alkoxyalkyl wie insbesondere Methoxyethyl und Ethoxyethyl,
Alkoxyalkenyl wie insbesondere 3-Methoxy-2-propenyl, Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, insbesondere 2-Propinyl und 2-Butinyl Halogenalkinyl wie insbesondere 3-Chlor-2-propinyl, sowie insbesondere die Reste 2-Cyanoethyl, Cyclopropylmethyl, 2-Cyclopropoxyethyl, 2-(2-Cyclopropoxyethoxy)ethyl, Methoxycarbonylmethyl, 2-Ethoxycarbonylethyl, N-Methylaminocarbonylmethyl, (2,2-Dimethylhydrazino)carbonylmethyl, Phenacyl, 3-Chlor-2-oxobutyl, 2-Methylthioethyl, 2-Ethylthio-1-methylethyl und 3-Mercaptopropyl bedeutet,
ein Rest $NR^7R^8$ worin
$R^7$,$R^8$ bevorzugt Phenyl, 4-Chlorphenyl, 4-Chlor-2-methylphenyl, Furyl, 3-Methoxycarbonylaminophenyl, 3-Methoxyphenyl, 4-Trifluoromethylphenyl, 4-(2-Chlorethoxy)phenyl, 2-Pyridyl, 3-Pyridyl, 2-Thienyl, Chloracetyl, Acetyl, Benzoyl, 2-Thienoyl, 2-Pyridylcarbonyl, 3-Chlorbenzoyl, 3-Dimethylamino-2-methylbenzoyl, 3-Methoxybenzoyl, 4-Trifluormethylbenzoyl und 5-Methylfuroyl sowie desweiteren Wasserstoff und/oder die im allgemeinen und im Besonderen bei $R^1$ genannte Alkyl-, Alkoxy-, Halogenalkyl- und Halogenalkoxygruppen bedeutet.

Die neuen Thiophenverbindungen I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.001 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.028 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.049 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 2.001 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 2.002 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 3.001 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.005 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 1.034 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 1,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |

| Botanischer Name | Deutscher Name |
|---|---|
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen Thiophenverbindungen I mit zahlreichen Vertretern anderer herbizider oder wachstumsregulierender Wirkstoffgruppen gemischt und gemeinsam ausgebracht werden. Beispielsweise kommen als Mischungspartner

Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt. Verbindungen ohne physikalische Daten lassen sich analog den Synthesebeispielen aus den entsprechenden Vorstufen herstellen. Sie lassen aufgrund ihrer hohen strukturellen Beziehung eine gleichartige Wirkung erwarten.

Beispiel 1

Zur Herstellung von 5′-Chlor-O-(2-butinyl)-2,2′-bithiophen-5-carbaldoxim der Formel

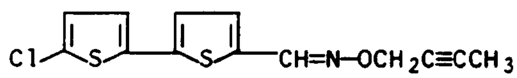

werden 2,0 g 5′-Chlor-2,2′-bithiophen-5-carbaldehyd mit 2,9 g Kaliumacetat in 30 ml 50 %igem Eisessig und 20 ml Isopropanol vorgelegt und mit 2,0 g O-(2-Butinyl)hydroxylammonium-oxalat versetzt. Man rührt 6 Stunden bei 60°C und läßt abkühlen. Nach Absaugen und Trocknen erhält man 3,3 g Kristallisat mit einem Schmelzpunkt von 104-107°C, das nach der Protonenresonanz-Spektroskopieaufnahme als E/Z-Gemisch im Verhältnis 3:1 vorliegt.

Beispiel 2

Entsprechend Beispiel 1 werden zur Herstellung von O-Ethyl-2,2′:5′,2″-terthiophen-5-carbaldoxim der Formel

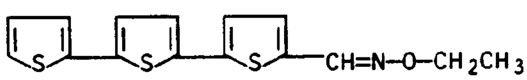

6,9 g des nach der Vorschrift in Heterocycles 24 (No. 3), 637-640 (1986) erhaltenen 2,2′:5′,2″-Terthiophen-5-carbaldehyds in 25 ml Methanol und 25 ml Methylenchlorid mit 2,2 g Natriumhydrogencarbonat und 2,44 g Ethoxyammonium-chlorid versetzt. Man rührt bei Raumtemperatur 10 Stunden, dampft ein und nimmt in Essigester auf. Man wäscht mit Wasser und gesättigter Bicarbonatlösung, trocknet und dampft i.V. ein. Der Rückstand wird aus Hexan umkristallisiert: 4,1 g braunes Pulver; Schmelzpunkt 49-51°C.

Tabelle 1.1

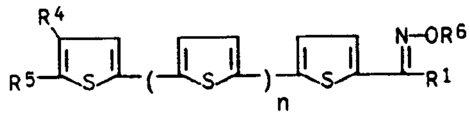

| Nr. | n | $R^5$ | $R^4$ | $R^1$ | $R^6$ | phys. Daten Fp (°C) |
|---|---|---|---|---|---|---|
| 1.001 | 1 | H | H | H | $-CH_2CH_3$ | 49-51 |
| 1.002 | 1 | H | H | H | $-CH_2CH=CH_2$ | 54-56 |
| 1.003 | 1 | H | H | H | $-CH_2CH=CHCl$ (trans) | 53-54 |
| 1.004 | 1 | H | H | H | $-CH_2CH=CHCH_3$ (trans) | 50-53 |
| 1.005 | 1 | H | H | H | $-CH(CH_3)C{\equiv}CH$ | 64-66 |
| 1.006 | 1 | H | H | $CH_3$ | $-CH_2CH_3$ | 61-66 |
| 1.007 | 1 | $CH_3$ | H | H | $-CH_2CH_3$ | |
| 1.008 | 1 | Br | H | H | $CH_3$ | |
| 1.009 | 1 | Cl | H | $CH_3$ | $CH_3$ | |
| 1.010 | 1 | H | H | H | $-CH_2CH_2OCH_3$ | |
| 1.011 | 1 | H | H | H | $-CH_2CH_2N(CH_3)_2$ | |
| 1.012 | 1 | H | H | H | $-CH_2CN$ | |

Tabelle 1.1 (Fortsetzung)

| Nr. | n | $R^5$ | $R^4$ | $R^1$ | $R^6$ | phys. Daten Fp (°C) |
|---|---|---|---|---|---|---|
| 1.013 | 1 | H | H | H | $-CH_2CH(OCH_2CH_3)_2$ | |
| 1.014 | 1 | H | H | H | $-CH_2CH_2OCH_3$ | |
| 1.015 | 1 | H | H | H | $-CH_2CONH_2$ | |
| 1.016 | 1 | H | H | H | $-CH_2C{\equiv}CH$ | |
| 1.017 | 1 | H | H | H | $-CH(CH_3)CH_2OCH_3$ | |
| 1.018 | 1 | H | H | H | $-CH_2CH_2Br$ | |
| 1.019 | 1 | Cl | H | H | $-(CH_2)_2CH_2$ | |
| 1.020 | 0 | H | H | H | $-CH_2CH{=}CH_2$ | Öl |
| 1.021 | 0 | Cl | H | H | $-CH_2CCl{=}CH_2$ | 53-56 |
| 1.022 | 0 | Cl | H | H | $-CH_2CH{=}CH_2$ | Harz |
| 1.023 | 0 | H | H | H | $-CH_2CCl{=}CH_2$ | |
| 1.024 | 0 | H | H | $CH_3$ | $-CH_2CH{=}CH_2$ | Harz |
| 1.025 | 0 | H | H | $CH_2CH_3$ | $-CH_2CH_3$ | Öl |
| 1.026 | 0 | H | H | $CH_3$ | $-CH_2CH_3$ | Öl |
| 1.027 | 0 | H | H | $CH_2CH_3$ | $-CH_2CH{=}CH_2$ | Öl |
| 1.028 | 0 | Cl | H | H | $-CH_2CH{=}CHCl$ (cis) | 30-32 |
| 1.029 | 0 | Cl | H | H | $-CH_2CH_3$ | 55-58 |
| 1.030 | 0 | Cl | H | H | $-CH_2CH{=}CHCl$ (trans) | 77-81 |
| 1.031 | 0 | Cl | H | H | $-(CH_2)_2CH_3$ | Öl |
| 1.032 | 0 | Cl | H | H | $-CH(CH_3)C{\equiv}CH$ | 72-75 |
| 1.033 | 0 | Cl | H | H | $-CH_2-C{\equiv}CCH_3$ | 104-107 |
| 1.034 | 0 | Cl | H | H | $-CH_2-C{\equiv}CH$ | 88-92 |
| 1.035 | 0 | Cl | H | H | $-CH_2CH_2N(CH_3)_2$ | Öl |
| 1.036 | 0 | Cl | H | H | $-CH_2CHCl{=}CH_2$ | 63-66 |
| 1.037 | 0 | Cl | H | H | $-CH_2$ 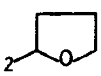 | Öl |
| 1.038 | 0 | Cl | H | H | $-CH_3$ | 92-94 |
| 1.039 | 0 | $CH_3$ | H | H | $-CH_2C{\equiv}CH$ | Öl |
| 1.040 | 0 | $CH_3$ | H | H | $-CH_2CH{=}CH_2$ | Öl |
| 1.041 | 0 | $CH_3$ | H | H | $-CH_2CH_3$ | Öl |
| 1.042 | 0 | I | H | H | $-CH_2CH{=}CH_2$ | Öl |
| 1.043 | 0 | $CH_3$ | H | H | $-CH_2CH_2N(CH_3)_2$ | |

Tabelle 1.1 (Fortsetzung)

| Nr. | n | $R^5$ | $R^4$ | $R^1$ | $R^6$ | phys. Daten Fp (°C) |
|---|---|---|---|---|---|---|
| 1.044 | 0 | $CH_2CH_3$ | H | H | $-CH_2CH_2N(CH_3)_2$ | |
| 1.045 | 0 | $CH_2CH_3$ | $CH_2CH_3$ | H | $-CH_2CH_2OCH_3$ | |
| 1.046 | 0 | Cl | Cl | H | $-CH_2CH_2OCH_3$ | |
| 1.047 | 0 | Cl | Cl | H | $-CH_2CH_2CH_2N(CH_3)_2$ | |
| 1.048 | 0 | $CH_3$ | $CH_3$ | H | $-CH_2C{\equiv}N$ | |
| 1.049 | 0 | Cl | H | H | $-CH_2CH_2Br$ | 70-73 |
| 1.050 | 0 | Cl | H | H | $-CH_2CH_2Cl$ | |
| 1.051 | 0 | Cl | H | H | $-CH_2CH_2OH$ | |
| 1.052 | 0 | $CH_3$ | H | H | $-CH_2COOCH_2CH_3$ | |
| 1.053 | 0 | $CH_3$ | H | H | $-CH_2CONH_2$ | |
| 1.054 | 0 | Cl | H | H | $-CH_2CONHN(CH_3)_2$ | |
| 1.055 | 0 | Cl | H | H | $-CH_2COOCH_3$ | |
| 1.056 | 0 | Cl | H | H | $-CH_2CH_2N(CH_3)_2$ | |
| 1.057 | 0 | Cl | H | H | $-CH_2CH_2OCH_2CH_3$ | |
| 1.058 | 0 | H | H | H | $-CH_2CH_2N(CH_3)_2$ | |
| 1.059 | 0 | Cl | H | H | $-CH_2CH_2N(CH_3)_2$ | |
| 1.060 | 0 | $CH_2CH_3$ | H | H | $-CH_2CH_2NH_2$ | |
| 1.061 | 0 | Cl | H | H | $-CH_2CH_2NHCH_2CH_3$ | |
| 1.062 | 0 | Cl | H | H | $-CH_2CH_2NHCHO$ | |
| 1.063 | 0 | Cl | Cl | H | $-CH_2CH{=}CH_2$ | |
| 1.064 | $CH_3$ | $CH_3$ | H | H | $-CH_2CH_2COOH$ | |
| 1.065 | 0 | $CH_2CH_3$ | H | H | $-CH_2CH_2NHCH_3$ | |
| 1.066 | 0 | Cl | Cl | H | $-CH_2CH_2OCH_3$ | |
| 1.067 | 0 | Cl | Cl | $CH_3$ | $-CH_2CH_2NHCH_3$ | |
| 1.068 | 0 | Cl | H | H | $-CH_2CH_2SH$ | |
| 1.069 | 0 | Cl | H | H | $-CH_2CH_2SCH_3$ | |
| 1.070 | 0 | Cl | H | H | $-CH_2CH_2SCH_2CH_3$ | |
| 1.071 | 0 | Cl | Cl | H | $-CH_2CH_2S(CH_2)_2CH_3$ | |
| 1.072 | 0 | Cl | H | H | $-CH_2NHCOCH_3$ | |
| 1.073 | 0 | Cl | H | H | $-CH_2CH_2COCH_3$ | |
| 1.074 | 0 | Cl | H | H | $-CH_2CH_2COCH_2Cl$ | |
| 1.075 | 0 | Cl | H | H | $-CH_2CH_2CH_2OCH_3$ | |
| 1.076 | 0 | Cl | H | H | $-CH_2CH_2O$ 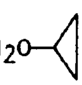 | |
| 1.077 | 0 | Cl | H | H | $-CH_2CH_2CH{=}CHCl$ | |

Tabelle 1.2

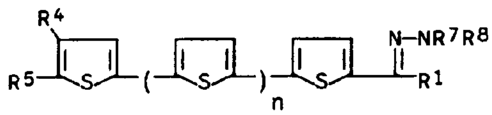

| Nr. | n | $R^5$ | $R^4$ | $R^1$ | $R^7$ | $R^8$ | phys. Daten Fp (°C) |
|---|---|---|---|---|---|---|---|
| 2.001 | 1 | H | H | H | H | $-CONH_2$ | 220 |
| 2.002 | 1 | H | H | H | H | Phenyl | 183-186 |
| 2.003 | 1 | H | H | H | H | 4-F-Phenyl | |
| 2.004 | 1 | H | H | H | Me | $-COCH_3$ | |
| 2.005 | 1 | H | H | H | H | $-COCH_3$ | |
| 2.006 | 1 | H | H | H | Me | $-COCH_2Cl$ | |
| 2.007 | 1 | H | H | H | H | $-COOCH_2CH_3$ | |
| 2.008 | 1 | H | H | H | H | $-COCH_3$ | |
| 2.009 | 1 | H | H | H | Me | -CHO | |
| 2.010 | 1 | H | H | H | Me | Methyl | |
| 2.011 | 1 | H | H | H | Et | Ethyl | |
| 2.012 | 0 | Cl | H | H | Et | Ethyl | |
| 2.013 | 0 | Cl | Cl | H | Me | $-CHOCH_2Cl$ | |
| 2.014 | 0 | Me | Me | H | H | $-COCH_2N(CH_3)_2$ | |
| 2.015 | 0 | Cl | H | H | H | $-COCH_2N(CH_3)_2$ | |

Tabelle 1.3

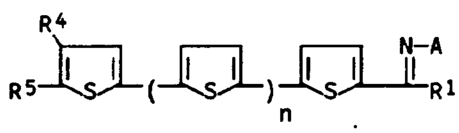

| Nr. | n | $R^5$ | $R^4$ | $R^1$ | A | phys. Daten Fp (°C) |
|---|---|---|---|---|---|---|
| 3.001 | 1 | H | H | H | Ph | 157-159 |
| 3.002 | 1 | H | H | H | 4-Cl-Phenyl | |
| 3.003 | 1 | H | H | H | 4-F-Phenyl | |
| 3.004 | 1 | H | H | H | $4-CH_3$-Phenyl | |
| 3.005 | 1 | H | H | H | 2-Pyridyl | |
| 3.006 | 1 | H | H | Me | 2-(5-Methylpyridyl) | |
| 3.007 | 1 | H | H | H | 2-Thienyl | |
| 3.008 | 1 | H | H | H | 5-(1,2,4-Triazolyl) | |

Tabelle 1.3 (Fortsetzung)

| Nr. | n | $R^5$ | $R^4$ | $R^1$ | A | phys. Daten Fp (°C) |
|---|---|---|---|---|---|---|
| 3.009 | 1 | H | H | H | 5-(2-Methyl-1,3-oxazolyl) oder 2-Methyl-1,3-oxazol-5-yl | |
| 3.010 | 1 | H | H | H | 5-(2-Ethyl-1,3-oxazyl) | |
| 3.011 | 1 | H | H | H | 2-Cl-Phenyl | |
| 3.012 | 0 | Cl | H | H | 5-(1,2,4-Triazyl) | |
| 3.013 | 0 | H | H | H | 5-(1,2,4-Triazyl) | 166-168 |
| 3.014 | 0 | Cl | H | Me | 5-(1,2,4-Triazyl) | |

Anwendungsbeispiele

Die herbizide Wirkung der Thiophenverbindungen der Formel I ließ sich durch Gewächshausversuche zeigen:

Zur Aufzucht der Testpflanzen dienten Plastikblumentöpfe mit 300 $cm^3$ Inhalt und lehmigem Sand mit etwa 3 % Humus als Substrat. Die Samen der Testpflanzen wurden nach Arten getrennt flach eingesät.

Bei Vorauflaufbehandlung wurden die aufbereiteten Wirkstoffe unmittelbar danach auf die Erdoberfläche aufgebracht. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen. Danach wurden die Gefäße mit durchsichtigen Plastikhauben abgedeckt, bis die Pflanzen angewaschsen waren. Diese Abdeckung fördert ein gleichmäßiges Keimen der Testpflanzen, sofern dies nicht durch die Wirkstoffe beeinträchtigt wird.

Zum Zwecke der Nachauflaufbehandlung wurden die Testpflanzen je nach Wuchsform erst bis zu einer Wuchshöhe von 3 bis 15 cm angezüchtet und erst dann mit den in Wasser suspendierten oder emulgierten Wirkstoffen behandelt. Die Testpflanzen wurden dafür entweder direkt gesät und in den gleichen Gefäßen aufgezogen oder sie wurden als Keimpflanzen getrennt gezüchtet und einige Tage vor der Behandlung in die Versuchsgefäße verpflanzt. Die Aufwandmenge für die Nachauflaufbehandlung betrug 0,25 kg Wirkstoff/ha. Eine Abdeckung unterblieb bei der Nachauflaufbehandlung.

Die Versuchsgefäße wurden im Gewächshaus aufgestellt, wobei für wärmeliebende Arten wärmere Bereiche (20 bis 35°C) und für solche gemäßigter Klimate 10 bis 25°C bevorzugt wurden. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürzung | Latein. Name | Deutscher Name | Englischer Name |
|---|---|---|---|
| ABUTH | Abutilon theophrasti | chinesischer Hanf | velvet leaf |
| AMARE | Amaranthus retroflexus | Zurückgekrümmter Fuchsschwanz | redroot pigweed |
| CHEAL | Chenopodium album | Weißer Gänsefuß | lambsquarters |
| DATST | Datura stramonium | Gemeiner Stechapfel | jimsonweed |
| LAMAM | Lamium amplexicaule | stengelumfassende Taubnessel | dead-nettle |
| ORYSA | Oryza sativa | Reis | rice |
| SOLNI | Solanum nigrum | Schwarzer Nacht-schatten | black nightshade |
| STEME | Stellaria media | Vogelsternmiere | chickweed |
| VERSS | Veronica spp. | Ehrenpreisarten | speedwell |

Mit 0,25 kg/ha a.S. im Nachauflaufverfahren eingesetzt, lassen sich mit den Verbindungen 1.001, 1.002 und 1.035 unerwünschte Pflanzen sehr gut bekämpfen. Reis erlitt keine oder nur geringfügige Schäden.

Verbindung Nr. 1.001 hat mit 0,25 kg/ha a.S. bei Nachauflaufanwendung herbizide Wirkung gegen Abutilon theophrasti, Datura stramonium und Lamium amplexicaule.

## Patentansprüche

**Pantentansprüche für folgende Vertragsstaaten : CH, DE, FR, GB, IT, LI, NL**

1. Thiophenverbindungen der allgemeinen Formel I

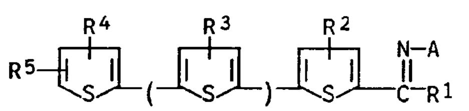

(I),

in der die Substituenten und der Index folgende Bedeutung haben:
n für 0 oder 1;
$R^1$ Wasserstoff, ein Halogenatom, eine $C_1$-$C_8$-Alkylgruppe, eine $C_1$-$C_6$-Alkoxygruppe, eine $C_1$-$C_8$-Halogenalkylgruppe oder eine $C_1$-$C_6$-Halogenalkoxygruppe ;
$R^2$, $R^3$, $R_4$ und $R^5$ Cyano, Nitro oder die bei $R^1$ genannten Gruppen;
A Wasserstoff, eine $C_1$-$C_8$-Alkylgruppe, eine $C_1$-$C_6$-Halogenalkylgruppe, eine $C_1$-$C_8$-Alkoxygruppe, eine $C_1$-$C_6$-Halogenalkoxygruppe, eine Arylgruppe oder eine Heteroarylgruppe, wobei die aromatischen Gruppen ein bis fünf Halogenatome und/oder ein bis drei der folgenden Reste tragen können: $C_1$-$C_8$-Alkyl, $C_1$-$C_6$-Halogenalkyl, $C_1$-$C_8$-Alkoxy, $C_1$-$C_6$-Halogenalkoxy, Hydroxycarbonylamino und/oder $C_1$-$C_4$-Alkoxycarbonylamino;
ein Rest $OR^6$, worin
$R^6$ Wasserstoff, $C_1$-$C_8$-Alkyl, $C_1$-$C_4$-Alkyl, welches ein- bis fünffach durch Halogen und/oder einfach durch einen der folgenden Reste substituiert ist: Cyano, $C_3$-$C_7$-Cycloalkyl, Hydroxy, $C_1$-$C_4$-Alkoxy, $C_1$-$C_4$-Alkylcarbonyl-$C_1$-$C_4$-alkoxy, $C_3$-$C_7$-Cycloalkylcarbonyl-$C_1$-$C_4$-alkoxy, $C_1$-$C_8$-Alkylcarbonyl, $C_1$-$C_8$-Halogenalkylcarbonyl, $C_3$-$C_8$-Cycloalkylcarbonyl, $C_1$-$C_8$-Alkoxycarbonyl, Mercapto, $C_1$-$C_4$-Alkylthio, Amino, $C_1$-$C_6$-Alkylamino, Di-$C_1$-$C_6$-Alkylamino, wobei diese Alkylgruppen auch gemeinsam mit dem Stickstoffatom einen aliphatischen Ring bilden können, Amino-$C_1$-$C_4$-alkoxy, $C_1$-$C_4$-Alkylamino-$C_1$-$C_4$-alkoxy, Di-$C_1$-$C_4$-Alkylamino-$C_1$-$C_4$-alkoxy, wobei die Alkylgruppen auch gemeinsam mit dem Stickstoffatom einen aliphatischen Ring bilden können, oder fünf- oder sechsgliedriges Heteroaryl, enthaltend ein bis drei der Heteroatome Stickstoff, Sauerstoff und/oder Schwefel, wobei dieser Cyclus seinerseits ein- bis fünffach durch Halogen und/oder ein- bis dreifach durch folgende Reste substituiert sein kann: $C_1$-$C_4$-Alkyl, $C_1$-$C_4$-Alkoxy, $C_1$-$C_4$-Halogenalkyl und/oder $C_1$-$C_4$-Halogenalkoxy; $C_3$-$C_9$-Cycloalkyl; $C_2$-$C_8$-Alkenyl, welches ein- bis fünffach durch Halogen und/oder einfach durch $C_1$-$C_4$-Alkoxy substituiert sein kann; $C_3$-$C_7$-Alkinyl, welches ein- bis fünffach durch Halogen und/oder einfach durch

$C_1$-$C_4$-Alkoxy substituiert sein kann;

bedeutet;

oder ein Rest $NR^7R^8$, worin

$R^7$, $R^8$ Wasserstoff, $C_1$-$C_8$-Alkyl, $C_1$-$C_8$-Alkoxy, $C_1$-$C_6$-Halogenalkyl, $C_1$-$C_6$-Halogenalkoxy, Aryl oder Hetaryl, die ein- bis dreifach duch Halogen, $C_1$-$C_8$-Alkyl, $C_1$-$C_8$-Alkoxy, $C_1$-$C_6$-Halogenalkyl, $C_1$-$C_6$-Halogenalkoxy oder Alkoxycarbonylamino substituiert sein können, $C_1$-$C_{12}$-Alkylcarbonyl, $C_1$-$C_{12}$-Halogenalkylcarbonyl, Aroyl oder Hetaroyl, welche ein- bis bis dreifach durch Halogen, $C_1$-$C_5$-Alkoxy, $C_1$-$C_4$-Halogenalkyl oder $C_1$-$C_6$-Alkyl substituiert sein können bedeutet,

sowie deren umweltverträgliche Salze;

mit der Maßgabe, daß, wenn n für 0 steht,

a) A keine ggf. substituierte Aryl- oder Heteroarylgruppe bedeutet, wenn $R^4$ Wasserstoff und gleichzeitig $R^5$ wasserstofff, Methyl oder Nitro bedeuten und

b) $R^7$ oder $R^8$ nicht für Phenyl oder substituiertes Phenyl steht und $R^7$ und $R^8$ nicht gleichzeitig Phenyl bedeuten, wenn $R^1$, $R^2$, $R^4$ und $R^5$ Wasserstoff bedeuten.

2. Thiophenverbindungen der Formel I gemäß Anspruch 1, in der n für 0 steht.

3. Verfahren zur Herstellung von Verbindungen der Formel I nach den Ansprüchen 1 oder 2, dadurch gekennzeichnet, daß man eine entsprechende Verbindung II

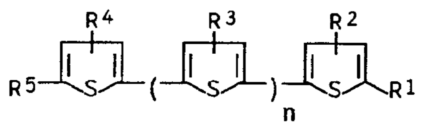

II,

in an sich bekannter Weise mit einer entsprechenden Hydroxylamin- oder Hydrazinverbindung oder einem primären Amin umsetzt.

4. Herbizide Mittel, enthaltend ein Thiophenderivat der Formel I gemäß den Ansprüchen 1 und 2 und inerte Zusatzstoffe.

5. Herbizide Mittel gemäß Anspruch 4, enthaltend ein Thiophenderivat der Formel I und weitere wirksame Bestandteile.

6. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Thiophenderivats der Formel I

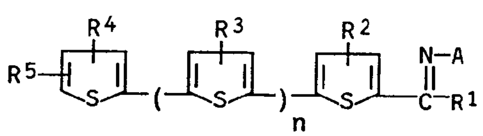

(I),

in der die Substituenten und der Index folgende Bedeutung haben:

n für 0 oder 1;

$R^1$ Wasserstoff, ein Halogenatom, eine $C_1$-$C_8$-Alkylgruppe, eine $C_1$-$C_6$-Alkoxygruppe, eine $C_1$-$C_8$-Halogenalkylgruppe oder eine $C_1$-$C_6$-Halogenalkoxygruppe;

$R^2$, $R^3$, $R^4$ und $R^5$ Cyano, Nitro oder die bei $R^1$ genannten Gruppen;

A Wasserstoff, eine $C_1$-$C_8$-Alkylgruppe, eine $C_1$-$C_6$-Halogenalkylgruppe, eine $C_1$-$C_8$-Alkoxygruppe, eine $C_1$-$C_6$-Halogenalkoxygruppe, eine Arylgruppe oder eine Heteroarylgruppe, wobei die aromatischen Gruppen ein bis fünf Halogenatome und/oder ein bis drei der folgenden Reste tragen können: $C_1$-$C_8$-Alkyl, $C_1$-$C_6$-Halogenalkyl, $C_1$-$C_8$-Alkoxy, $C_1$-$C_6$-Halogenalkoxy, Hydroxycarbonylamino und/oder $C_1$-$C_4$-Alkoxycarbonylamino;

ein Rest $OR^6$, worin

$R^6$ Wasserstoff, $C_1$-$C_8$-Alkyl, $C_1$-$C_4$-Alkyl, welches ein- bis fünffach durch Halogen und/oder einfach durch einen der folgenden Reste substituiert ist: Cyano, $C_3$-$C_7$-Cycloalkyl, hydroxy $C_1$-$C_4$-Alkoxy, $C_1$-$C_4$-Alkylcarbonyl-$C_1$-$C_4$-alkoxy, $C_3$-$C_7$-Cycloalkylcarbonyl-$C_1$-$C_4$-alkoxy, $C_1$-$C_8$-Alkylcarbonyl-$C_1$-$C_8$-Halogenalkylcarbonyl, $C_3$-$C_8$-Cycloalkylcarbonyl, $C_1$-$C_8$-Alkoxycarbonyl, Mercapto, $C_1$-$C_4$-Alkylthio, Amino, $C_1$-$C_6$-Alkylamino, Di-$C_1$-$C_6$-Alkylamino, wobei diese Alkylgruppen auch gemeinsam mit dem Stickstoffatom einen aliphatischen Ring bilden können, Amino-$C_1$-$C_4$-alkoxy, $C_1$-$C_4$-Alkylamino-$C_1$-$C_4$-alkoxy, Di-$C_1$-$C_4$-Alkylamino-$C_1$-$C_4$-alkoxy, wobei die Alkylgruppen auch gemeinsam mit dem Stickstoffatom einen aliphatischen Ring bilden können, oder fünf- oder sechsgliedriges Heteroaryl, enthaltend ein bis drei der Heteroatome Stickstoff, Sauerstoff und/oder

Schwefel, wobei dieser Cyclus seinerseits ein- bis fünffach durch Halogen und/oder ein- bis dreifach durch folgende Reste substituiert sein kann: $C_1$-$C_4$-Alkyl, $C_1$-$C_4$-Alkoxy, $C_1$-$C_4$-Halogenalkyl und/oder $C_1$-$C_4$-Halogenalkoxy; $C_3$-$C_9$-Cycloalkyl; $C_2$-$C_8$-Alkenyl, welches ein- bis fünffach durch Halogen und/oder einfach durch $C_1$-$C_4$-Alkoxy substituiert sein kann; $C_3$-$C_7$-Alkinyl, welches ein- bis fünffach durch Halogen und/oder einfach durch $C_1$-$C_4$-Alkoxy substituiert sein kann; bedeutet;

oder ein Rest $NR^7R^8$, worin

$R^7$, $R^8$ Wasserstoff, $C_1$-$C_8$-Alkyl, $C_1$-$C_8$-Alkoxy, $C_1$-$C_6$-Halogenalkyl, $C_1$-$C_6$-Halogenalkoxy, Aryl oder Hetaryl, die ein- bis dreifach duch Halogen, $C_1$-$C_8$-Alkyl, $C_1$-$C_8$-Alkoxy, $C_1$-$C_6$-Halogenalkyl, $C_1$-$C_6$-Halogenalkoxy oder Alkoxycarbonylamino substituiert sein können, $C_1$-$C_{12}$-Alkylcarbonyl, $C_1$-$C_{12}$- Halogenalkylcarbonyl, Aroyl oder Hetaroyl, welche ein- bis bis dreifach durch Halogen, $C_1$-$C_5$-Alkoxy, $C_1$-$C_4$-Halogenalkyl oder $C_1$-$C_6$-Alkyl substituiert sein können bedeutet,

oder deren umweltverträglichen Salzen behandelt.

7. Verwendung einer Verbindung I nach Anspruch 6 als Herbizid.

## Pantentansprüche für folgende Vertragsstaat : ES

1. Verfahren zur Herstellung von Thiophenverbindungen der allgemeinen Formel I

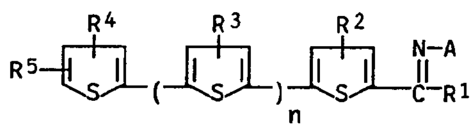 (I),

in der die Substituenten und der Index folgende Bedeutung haben:

n für 0 oder 1;

$R^1$ Wasserstoff, ein Halogenatom, eine $C_1$-$C_8$-Alkylgruppe, eine $C_1$-$C_6$-Alkoxygruppe, eine $C_1$-$C_8$-Halogenalkylgruppe oder eine $C_1$-$C_6$-Halogenalkoxygruppe ;

$R^2$, $R^3$, $R^4$ und $R^5$ Cyano, Nitro oder die bei $R^1$ genannten Gruppen;

A Wasserstoff, eine $C_1$-$C_8$-Alkylgruppe, eine $C_1$-$C_6$-Halogenalkylgruppe, eine $C_1$-$C_8$-Alkoxygruppe, eine $C_1$-$C_6$-Halogenalkoxygruppe, eine Arylgruppe oder eine Heteroarylgruppe, wobei die aromatischen Gruppen ein bis fünf Halogenatome und/oder ein bis drei der folgenden Reste tragen können: $C_1$-$C_8$-Alkyl, $C_1$-$C_6$-Halogenalkyl, $C_1$-$C_8$-Alkoxy, $C_1$-$C_6$-Halogenalkoxy, Hydroxycarbonylamino und/oder $C_1$-$C_4$-Alkoxycarbonylamino;

ein Rest $OR^6$, worin

$R^6$ Wasserstoff, $C_1$-$C_8$-Alkyl, $C_1$-$C_4$-Alkyl, welches ein- bis fünffach durch Halogen und/oder einfach durch einen der folgenden Reste substituiert ist: Cyano, $C_3$-$C_7$-Cycloalkyl, Hydroxy, $C_1$-$C_4$-Alkoxy, $C_1$-$C_4$-Alkylcarbonyl-$C_1$-$C_4$-alkoxy, $C_3$-$C_7$-Cycloalkylcarbonyl-$C_1$-$C_4$-alkoxy, $C_1$-$C_8$-Alkylcarbonyl, $C_1$-$C_8$-Halogenalkylcarbonyl, $C_3$-$C_8$-Cycloalkylcarbonyl, $C_1$-$C_8$-Alkoxycarbonyl, Mercapto, $C_1$-$C_4$-Alkylthio, Amino, $C_1$-$C_6$-Alkylamino, Di-$C_1$-$C_6$-Alkylamino, wobei diese Alkylgruppen auch gemeinsam mit dem Stickstoffatom einen aliphatischen Ring bilden können, Amino-$C_1$-$C_4$-alkoxy, $C_1$-$C_4$-Alkylamino-$C_1$-$C_4$-alkoxy, Di-$C_1$-$C_4$-Alkylamino-$C_1$-$C_4$-alkoxy, wobei die Alkylgruppen auch gemeinsam mit dem Stickstoffatom einen aliphatischen Ring bilden können, oder fünf- oder sechsgliedriges Heteroaryl, enthaltend ein bis drei der Heteroatome Stickstoff, Sauerstoff und/oder Schwefel, wobei dieser Cyclus seinerseits ein- bis fünffach durch Halogen und/oder ein- bis dreifach durch folgende Reste substituiert sein kann: $C_1$-$C_4$-Alkyl, $C_1$-$C_4$-Alkoxy, $C_1$-$C_4$-Halogenalkyl und/oder $C_1$-$C_4$-Halogenalkoxy; $C_3$-$C_9$-Cycloalkyl; $C_2$-$C_8$-Alkenyl, welches ein- bis fünffach durch Halogen und/oder einfach durch $C_1$-$C_4$-Alkoxy substituiert sein kann; $C_3$-$C_7$-Alkinyl, welches ein- bis fünffach durch Halogen und/oder einfach durch $C_1$-$C_4$-Alkoxy substituiert sein kann;

bedeutet;

oder ein Rest $NR^7R^8$, worin

$R^7$, $R^8$ Wasserstoff, $C_1$-$C_8$-Alkyl, $C_1$-$C_8$-Alkoxy, $C_1$-$C_6$-Halogenalkyl, $C_1$-$C_6$-Halogenalkoxy, Aryl oder Hetaryl, die ein- bis dreifach duch Halogen, $C_1$-$C_8$-Alkyl, $C_1$-$C_8$-Alkoxy, $C_1$-$C_6$-Halogenalkyl, $C_1$-$C_6$-Halogenalkoxy oder Alkoxycarbonylamino substituiert sein können, $C_1$-$C_{12}$-Alkylcarbonyl, $C_1$-$C_{12}$- Halogenalkylcarbonyl, Aroyl oder Hetaroyl, welche ein- bis bis dreifach durch Halogen, $C_1$-$C_5$-Alkoxy, $C_1$-$C_4$-Halogenalkyl oder $C_1$-$C_6$-Alkyl substituiert sein können bedeutet,

sowie deren umweltverträgliche Salze,

mit der Maßgabe, daß, wenn n für 0 steht,

a) A keine ggf. substituierte Aryl- oder Heteroarylgruppe bedeutet, wenn $R^4$ Wasserstoff und gleichzeitig $R^5$ Wasserstoff, Methyl oder Nitro bedeuten und

b) $R^7$ oder $R^8$ nicht für Phenyl oder substituiertes Phenyl steht und $R^7$ und $R^8$ nicht gleichzeitig Phenyl bedeuten, wenn $R^1$, $R^2$, $R^4$ und $R^5$ Wasserstoff bedeuten,
dadurch gekennzeichnet, daß man eine entsprechende Verbindung II

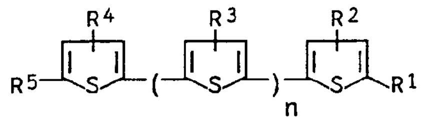

II,

in an sich bekannter Weise mit einer entsprechenden Hydroxylamin- oder Hydrazinverbindung oder einem primären Amin umsetzt.

2. Verfahren zur Herstellung von Thiophenverbindungen der Formel I gemäß Anspruch 1, in der n für 0 steht.

3. Herbizide Mittel, enthaltend ein Thiophenderivat der Formel I gemäß den Ansprüchen 1 und 2 und inerte Zusatzstoffe.

4. Herbizide Mittel gemäß Anspruch 3, enthaltend ein Thiophenderivat der Formel I und weitere wirksame Bestandteile.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Thiophenderivats der Formel I

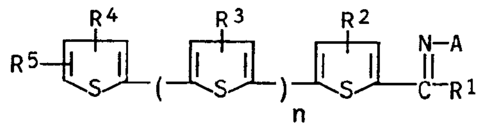

(I),

in der die Substituenten und der Index folgende Bedeutung haben:
n für 0 oder 1;
$R^1$ Wasserstoff, ein Halogenatom, eine $C_1$-$C_8$-Alkylgruppe, eine $C_1$-$C_6$-Alkoxygruppe, eine $C_1$-$C_8$-Halogenalkylgruppe oder eine $C_1$-$C_6$-Halogenalkoxygruppe ;
$R^2$, $R^3$, $R^4$ und $R^5$ Cyano, Nitro oder die bei $R^1$ genannten Gruppen;
A Wasserstoff, eine $C_1$-$C_8$-Alkylgruppe, eine $C_1$-$C_6$-Halogenalkylgruppe, eine $C_1$-$C_8$-Alkoxygruppe, eine $C_1$-$C_6$-Halogenalkoxygruppe, eine Arylgruppe oder eine Heteroarylgruppe, wobei die aromatischen Gruppen ein bis fünf Halogenatome und/oder ein bis drei der folgenden Reste tragen können: $C_1$-$C_8$-Alkyl, $C_1$-$C_6$-Halogenalkyl, $C_1$-$C_8$-Alkoxy, $C_1$-$C_6$-Halogenalkoxy, Hydroxycarbonylamino und/oder $C_1$-$C_4$-Alkoxycarbonylamino;
ein Rest $OR^6$, worin
$R^6$ Wasserstoff, $C_1$-$C_8$-Alkyl, $C_1$-$C_4$-Alkyl, welches ein- bis fünffach durch Halogen und/oder einfach durch einen der folgenden Reste substituiert ist: Cyano, $C_3$-$C_7$-Cycloalkyl, Hydroxy, $C_1$-$C_4$-Alkoxy, $C_1$-$C_4$-Alkylcarbonyl-$C_1$-$C_4$-alkoxy, $C_3$-$C_7$-Cycloalkylcarbonyl-$C_1$-$C_4$-alkoxy, $C_1$-$C_8$-Alkylcarbonyl, $C_1$-$C_8$-Halogenalkylcarbonyl, $C_3C_8$-Cycloalkylcarbonyl, $C_1$-$C_8$-Alkoxycarbonyl, Mercapto, $C_1$-$C_4$-Alkylthio, Amino, $C_1$-$C_6$-Alkylamino, Di-$C_1$-$C_6$-Alkylamino, wobei diese Alkylgruppen auch gemeinsam mit dem Stickstoffatom einen aliphatischen Ring bilden können, Amino-$C_1$-$C_4$-alkoxy, $C_1$-$C_4$-Alkylamino-$C_1$-$C_4$-alkoxy, Di-$C_1$-$C_4$-Alkylamino-$C_1$-$C_4$-alkoxy, wobei die Alkylgruppen auch gemeinsam mit dem Stickstoffatom einen aliphatischen Ring bilden können, oder fünf- oder sechsgliedriges Heteroaryl, enthaltend ein bis drei der Heteroatome Stickstoff, Sauerstoff und/oder Schwefel, wobei dieser Cyclus seinerseits ein- bis fünffach durch Halogen und/oder ein- bis dreifach durch folgende Reste substituiert sein kann: $C_1$-$C_4$-Alkyl, $C_1$-$C_4$-Alkoxy, $C_1$-$C_4$-Halogenalkyl und/oder $C_1$-$C_4$-Halogenalkoxy;$C_3$-$C_9$-Cycloalkyl; $C_2$-$C_8$-Alkenyl, welches ein- bis fünffach durch Halogen und/oder einfach durch $C_1$-$C_4$-Alkoxy substituiert sein kann; $C_3$-$C_7$-Alkinyl, welches ein- bis fünffach durch Halogen und/oder einfach durch $C_1$-$C_4$-Alkoxy substituiert sein kann;
bedeutet;
oder ein Rest $NR^7R^8$, worin
$R^7$, $R^8$ Wasserstoff, $C_1$-$C_8$-Alkyl, $C_1$-$C_8$-Alkoxy, $C_1$-$C_6$-Halogenalkyl, $C_1$-$C_6$-Halogenalkoxy, Aryl oder Hetaryl, die ein- bis dreifach duch Halogen, $C_1$-$C_8$-Alkyl, $C_1$-$C_8$-Alkoxy, $C_1$-$C_6$-Halogenalkyl, $C_1$-$C_6$-Halogenalkoxy oder Alkoxycarbonylamino substituiert sein können, $C_1$-$C_{12}$-Alkylcarbonyl, $C_1$-$C_{12}$- Halogenalkylcarbonyl, Aroyl oder Hetaroyl, welche ein- bis bis dreifach durch Halogen, $C_1$-$C_5$-Alkoxy, $C_1$-$C_4$-Halogenalkyl oder $C_1$-$C_6$-Alkyl substituiert sein können bedeutet,

oder deren umweltverträglichen Salzen behandelt.

6. Verwendung einer Verbindung I nach Anspruch 5 als Herbizid.

## Claims

### Claims for the Following Contracting States : CH, DE, FR, GB, IT, LI, NL

1. A thiophene compound of the formula I

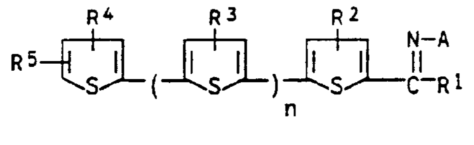

(I)

where n is 0 or 1;

$R^1$ is hydrogen, halogen, $C_1$-$C_8$-alkyl, $C_1$-$C_6$-alkoxy, $C_1$-$C_8$-haloalkyl or $C_1$-$C_6$-haloalkoxy;

$R^2$, $R^3$, $R^4$ and $R^5$ are each cyano, nitro or the groups stated for $R^1$;

A is hydrogen, $C_1$-$C_8$-alkyl, $C_1$-$C_6$-haloalkyl, $C_1$-$C_8$-alkoxy, $C_1$-$C_6$-haloalkoxy, aryl or hetaryl, and the aromatic groups may carry from one to five halogen atoms and/or from one to three of the following radicals: $C_1$-$C_8$-alkyl, $C_1$-$C_6$-haloalkyl,$C_1$-$C_8$-alkoxy, $C_1$-$C_6$-haloalkoxy,hydroxycarbonylamino and/or $C_1$-$C_4$-alkoxycarbonylamino;

a radical $OR^6$, where

$R^6$ is hydrogen, $C_1$-$C_8$-alkyl, $C_1$-$C_4$-alkyl which is mono-substituted to pentasubstituted by halogen and/or mono-substituted by one of the following radicals: cyano, $C_3$-$C_7$-cycloalkyl, hydroxyl, $C_1$-$C_4$-alkoxy, $C_1$-$C_4$-alkylcarbonyl-$C_1$-$C_4$-alkoxy, $C_3$-$C_7$-cycloalkylcarbonyl-$C_1$-$C_4$-alkoxy, $C_1$-$C_8$-alkylcarbonyl, $C_1$-$C_8$-haloalkylcarbonyl, $C_3$-$C_8$-cycloalkylcarbonyl, $C_1$-$C_8$-alkoxycarbonyl, mercapto, $C_1$-$C_4$-alkylthio, amino, $C_1$-$C_6$-alkylamino or di-$C_1$-$C_6$-alkylamino, where these alkyl groups together with the nitrogen atom may furthermore form an aliphatic ring, or is amino-$C_1$-$C_4$-alkoxy, $C_1$-$C_4$-alkylamino-$C_1$-$C_4$-alkoxy or di-$C_1$-$C_4$-alkylamino-$C_1$-$C_4$-alkoxy, where the alkyl groups together with the nitrogen atom may furthermore form an aliphatic ring, or five-membered or six-membered hetaryl containing from one to three of the heteroatoms nitrogen, oxygen and/or sulfur, where this cyclic structure in turn may be mono-substituted to pentasubstituted by halogen and/or mono-substituted to trisubstituted by the following radicals: $C_1$-$C_4$-alkyl, $C_1$-$C_4$-alkoxy, $C_1$-$C_4$-haloalkyl and/or $C_1$-$C_4$-haloalkoxy; $C_3$-$C_9$-cycloalkyl; $C_2$-$C_8$-alkenyl which may be monosubstituted to pentasubstituted by halogen and/or monosubstituted by $C_1$-$C_4$-alkoxy; $C_3$-$C_7$-alkynyl which may be mono-substituted to pentasubstituted by halogen and/or mono-substituted by $C_1$-$C_4$-alkoxy;

or a radical $NR^7R^8$, where

$R^7$ and $R^8$ are each hydrogen, $C_1$-$C_8$-alkyl, $C_1$-$C_8$-alkoxy, $C_1$-$C_6$-haloalkyl, $C_1$-$C_6$-haloalkoxy, aryl or hetaryl which may be mono-substituted to trisubstituted by halogen, $C_1$-$C_8$-alkyl, $C_1$-$C_8$-alkoxy, $C_1$-$C_6$-haloalkyl, $C_1$-$C_6$-haloalkoxy or alkoxycarbonylamino, or are each $C_1$-$C_6$-alkylcarbonyl, $C_1$-$C_{12}$-haloalkylcarbonyl, aroyl or hetaroyl which may be monosubstituted to trisubstituted by halogen, $C_1$-$C_5$-alkoxy, $C_1$-$C_4$-haloalkyl or $C_1$-$C_6$-alkyl, and environmentally compatible salts thereof;

with the proviso that, if n is 0,

a) A is not unsubstituted or substituted aryl or hetaryl if $R^4$ is hydrogen and simultaneously $R^5$ is hydrogen, methyl or nitro and

b) $R^7$ or $R^8$ is not phenyl or substituted phenyl and $R^7$ and $R^8$ are not simultaneously phenyl if $R^1$, $R^2$, $R^4$ and $R^5$ are hydrogen.

2. A thiophene compound of the formula I as claimed in claim 1, where n is 0.

3. A process for the preparation of a compound of the formula I as claimed in either of claims 1 and 2, wherein a corresponding compound II

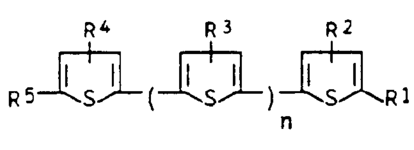

(II)

is reacted in a conventional manner with a corresponding hydroxylamine or hydrazine compound or with a primary amine.

4. A herbicide, containing a thiophene derivative of the formula I as claimed in either of claims 1 and 2 and inert additives.

5. A herbicide as claimed in claim 4, containing a thiophene derivative of the formula I and further active ingredients.

6. A method for controlling undesirable plant growth, wherein the undesirable plants and/or their habitat are or is treated with a herbicidally effective amount of a thiophene derivative of the formula I

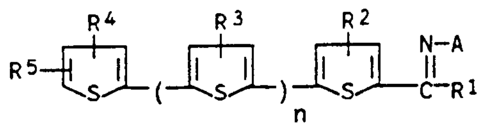

(I)

where n is 0 or 1;

$R^1$ is hydrogen, halogen, $C_1$-$C_8$-alkyl, $C_1$-$C_6$-alkoxy, $C_1$-$C_8$-haloalkyl or $C_1$-$C_6$-haloalkoxy;

$R^2$, $R^3$, $R^4$ and $R^5$ are each cyano, nitro or the groups stated for $R^1$;

A is hydrogen, $C_1$-$C_8$-alkyl, $C_1$-$C_6$-haloalkyl, $C_1$-$C_8$-alkoxy, $C_1$-$C_6$-haloalkoxy, aryl or hetaryl, and the aromatic groups may carry from one to five halogen atoms and/or from one to three of the following radicals: $C_1$-$C_8$-alkyl, $C_1$-$C_6$-haloalkyl, $C_1$-$C_8$-alkoxy, $C_1$-$C_6$-haloalkoxy, hydroxycarbonylamino and/or $C_1$-$C_4$-alkoxycarbonylamino;

a radical $OR^6$, where

$R^6$ is hydrogen, $C_1$-$C_8$-alkyl, $C_1$-$C_4$-alkyl which is monosubstituted to pentasubstituted by halogen and/or monosubstituted by one of the following radicals: cyano, $C_3$-$C_7$-cycloalkyl, hydroxyl, $C_1$-$C_4$-alkoxy, $C_1$-$C_4$-alkylcarbonyl-$C_1$-$C_4$-alkoxy, $C_3$-$C_7$-cycloalkylcarbonyl-$C_1$-$C_4$-alkoxy, $C_1$-$C_8$-alkylcarbonyl, $C_1$-$C_8$-haloalkylcarbonyl, $C_3$-$C_8$-cycloalkylcarbonyl, $C_1$-$C_8$-alkoxycarbonyl, mercapto, $C_1$-$C_4$-alkylthio, amino, $C_1$-$C_6$-alkylamino or di-$C_1$-$C_6$-alkylamino, where these alkyl groups together with the nitrogen atom may furthermore form an aliphatic ring, or is amino-$C_1$-$C_4$-alkoxy, $C_1$-$C_4$-alkylamino-$C_1$-$C_4$-alkoxy or di-$C_1$-$C_4$-alkylamino-$C_1$-$C_4$-alkoxy, where the alkyl groups together with the nitrogen atom may furthermore form an aliphatic ring, or five-membered or six-membered hetaryl containing from one to three of the heteroatoms nitrogen, oxygen and/or sulfur, where this cyclic structure in turn may be mono-substituted to pentasubstituted by halogen and/or mono-substituted to trisubstituted by the following radicals: $C_1$-$C_4$-alkyl, $C_1$-$C_4$-alkoxy, $C_1$-$C_4$-haloalkyl and/or $C_1$-$C_4$-haloalkoxy; $C_3$-$C_9$-cycloalkyl; $C_2$-$C_8$-alkenyl which may be monosubstituted to pentasubstituted by halogen and/or monosubstituted by $C_1$-$C_4$-alkoxy; $C_3$-$C_7$-alkynyl which may be monosubstituted to pentasubstituted by halogen and/or monosubstituted by $C_1$-$C_4$-alkoxy;

or a radical $NR^7R^8$, where

$R^7$ and $R^8$ are each hydrogen, $C_1$-$C_8$-alkyl, $C_1$-$C_8$-alkoxy, $C_1$-$C_6$-haloalkyl, $C_1$-$C_6$-haloalkoxy, aryl or hetaryl which may be monosubstituted to trisubstituted by halogen, $C_1$-$C_8$-alkyl, $C_1$-$C_6$-alkoxy, $C_1$-$C_6$-haloalkyl, $C_1$-$C_6$-haloalkoxy or alkoxycarbonylamino, or are each $C_1$-$C_{12}$-alkylcarbonyl, $C_1$-$C_{12}$-haloalkylcarbonyl, aroyl or hetaroyl which may be monosubstituted to trisubstituted by halogen, $C_1$-$C_5$-alkoxy, $C_1$-$C_4$-haloalkyl or $C_1$-$C_6$-alkyl, or environmentally compatible salts thereof.

7. Use of a compound I as claimed in claim 6 as a herbicide.

**Claims for the Following Contracting State : ES**

1. A process for the preparation of a thiophene compound of the formula I

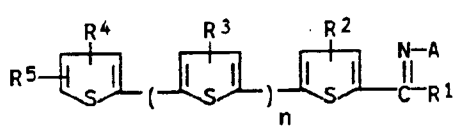

(I)

n is 0 or 1;

$R^1$ is hydrogen, halogen, $C_1$-$C_8$-alkyl, $C_1$-$C_6$-alkoxy, $C_1$-$C_8$-haloalkyl or $C_1$-$C_6$-haloalkoxy;

$R^2$, $R^3$, $R^4$ and $R^5$ are each cyano, nitro or the groups stated for $R^1$;

A is hydrogen, $C_1$-$C_8$-alkyl, $C_1$-$C_6$-haloalkyl, $C_1$-$C_8$-alkoxy, $C_1$-$C_6$-haloalkoxy, aryl or hetaryl, and the aromatic groups may carry from one to five halogen atoms and/or from one to three of the following radicals: $C_1$-$C_8$-alkyl, $C_1$-$C_6$-haloalkyl, $C_1$-$C_8$-alkoxy, $C_1$-$C_6$-haloalkoxy, hydroxycarbonylamino and/or $C_1$-$C_4$-alkoxycarbonylamino;

a radical $OR^6$, where

$R^6$ is hydrogen, $C_1$-$C_8$-alkyl, $C_1$-$C_4$-alkyl which is monosubstituted to pentasubstituted by halogen and/or monosubstituted by one of the following radicals: cyano, $C_3$-$C_7$-cycloalkyl, hydroxyl, $C_1$-$C_4$-alkoxy, $C_1$-$C_4$-alkylcarbonyl-$C_1$-$C_4$-alkoxy, $C_3$-$C_7$-cycloalkylcarbonyl-$C_1$-$C_4$-alkoxy, $C_1$-$C_8$-alkylcarbonyl, $C_1$-$C_8$-haloalkylcarbonyl, $C_3$-$C_8$-cycloalkylcarbonyl, $C_1$-$C_8$-alkoxycarbonyl, mercapto, $C_1$-$C_4$-alkylthio, amino, $C_1$-$C_6$-alkylamino or di-$C_1$-$C_6$-alkylamino, where these alkyl groups together with the nitrogen atom may furthermore form an aliphatic ring, or is amino-$C_1$-$C_4$-alkoxy, $C_1$-$C_4$-alkylamino-$C_1$-$C_4$-alkoxy or di-$C_1$-$C_4$-alkylamino-$C_1$-$C_4$-alkoxy, where the alkyl groups together with the nitrogen atom may furthermore form an aliphatic ring, or five-membered or six-membered hetaryl containing from one to three of the heteroatoms nitrogen, oxygen and/or sulfur, where this cyclic structure in turn may be mono-substituted to pentasubstituted by halogen and/or mono-substituted to trisubstituted by the following radicals: $C_1$-$C_4$-alkyl, $C_1$-$C_4$-alkoxy, $C_1$-$C_4$-haloalkyl and/or $C_1$-$C_4$-haloalkoxy; $C_3$-$C_9$-cycloalkyl; $C_2$-$C_8$-alkenyl which may be monosubstituted to pentasubstituted by halogen and/or monosubstituted by $C_1$-$C_4$-alkoxy; $C_3$-$C_7$-alkynyl which may be monosubstituted to pentasubstituted by halogen and/or monosubstituted by $C_1$-$C_4$-alkoxy;

or a radical $NR^7R^8$, where

$R^7$ and $R^8$ are each hydrogen, $C_1$-$C_8$-alkyl, $C_1$-$C_8$-alkoxy, $C_1$-$C_6$-haloalkyl, $C_1$-$C_6$-haloalkoxy, aryl or hetaryl which may be monosubstituted to trisubstituted by halogen, $C_1$-$C_8$-alkyl, $C_1$-$C_8$-alkoxy, $C_1$-$C_6$-haloalkyl, $C_1$-$C_6$-haloalkoxy or alkoxycarbonylamino, or are each $C_1$-$C_6$-alkylcarbonyl, $C_1$-$C_{12}$-haloalkylcarbonyl, aroyl or hetaroyl which may be monosubstituted to trisubstituted by halogen, $C_1$-$C_5$-alkoxy, $C_1$-$C_4$-haloalkyl or $C_1$-$C_6$-alkyl,

and environmentally compatible salts thereof,

with the proviso that, if n is 0,

a) A is not unsubstituted or substituted aryl or hetaryl if $R^4$ is hydrogen and simultaneously $R^5$ is hydrogen, methyl or nitro and

b) $R^7$ or $R^8$ is not phenyl or substituted phenyl and $R^7$ and $R^8$ are not simultaneously phenyl if $R^1$, $R^2$, $R^4$ and $R^5$ are hydrogen, wherein a corresponding compound II

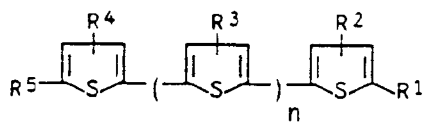

II

is reacted in a conventional manner with a corresponding hydroxylamine or hydrazine compound or with a primary amine.

2. A process for the preparation of a thiophene compound of the formula I as claimed in claim 1, where n is 0.

3. A herbicide, containing a thiophene derivative of the formula I, as claimed in either of claims 1 and 2 and inert additives.

4. A herbicide as claimed in claim 3, containing a thiophene derivative of the formula I and further active ingredients.

5. A method for controlling undesirable plant growth, wherein the undesirable plants and/or their habitat are or is treated with a herbicidally effective amount of a thiophene derivative of the formula I

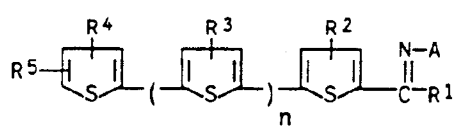

(I)

where n is 0 or 1;

$R^1$ is hydrogen, halogen, $C_1$-$C_8$-alkyl, $C_1$-$C_6$-alkoxy, $C_1$-$C_8$-haloalkyl or $C_1$-$C_6$-haloalkoxy;

$R^2$, $R^3$, $R^4$ and $R^5$ are each cyano, nitro or the groups stated for $R^1$;

A is hydrogen, $C_1$-$C_8$-alkyl, $C_1$-$C_6$-haloalkyl, $C_1$-$C_8$-alkoxy, $C_1$-$C_6$-haloalkoxy, aryl or hetaryl, and the aromatic groups may carry from one to five halogen atoms and/or from one to three of the following radicals: $C_1$-$C_8$-alkyl, $C_1$-$C_6$-haloalkyl, $C_1$-$C_8$-alkoxy, $C_1$-$C_6$-haloalkoxy, hydroxycarbonylamino and/or $C_1$-$C_4$-alkoxycarbonylamino;

a radical $OR^6$, where

$R^6$ is hydrogen, $C_1$-$C_8$-alkyl, $C_1$-$C_4$-alkyl which is monosubstituted to pentasubstituted by halogen and/or

monosubstituted by one of the following radicals: cyano, $C_3$-$C_7$-cycloalkyl, hydroxyl, $C_1$-$C_4$-alkoxy, $C_1$-$C_4$-alkylcarbonyl-$C_1$-$C_4$-alkoxy, $C_3$-$C_7$-cycloalkylcarbonyl-$C_1$-$C_4$-alkoxy, $C_1$-$C_8$-alkylcarbonyl, $C_1$-$C_8$-haloalkylcarbonyl, $C_3$-$C_8$-cycloalkylcarbonyl, $C_1$-$C_8$-alkoxycarbonyl, mercapto, $C_1$-$C_4$alkylthio, amino, $C_1$-$C_6$-alkylamino or di-$C_1$-$C_6$-alkylamino, where these alkyl groups together with the nitrogen atom may furthermore form an aliphatic ring, or is amino-$C_1$-$C_4$-alkoxy, $C_1$-$C_4$-alkylamino-$C_1$-$C_4$-alkoxy or di-$C_1$-$C_4$-alkylamino-$C_1$-$C_4$-alkoxy, where the alkyl groups together with the nitrogen atom may furthermore form an aliphatic ring, or five-membered or six-membered hetaryl containing from one to three of the heteroatoms nitrogen, oxygen and/or sulfur, where this cyclic structure in turn may be mono-substituted to pentasubstituted by halogen and/or mono-substituted to trisubstituted by the following radicals: $C_1$-$C_4$-alkyl, $C_1$-$C_4$-alkoxy, $C_1$-$C_4$-haloalkyl and/or $C_1$-$C_4$-haloalkoxy;$C_3$-$C_8$-cycloalkyl; $C_3$-$C_8$-alkenyl which may be monosubstituted to pentasubstituted by halogen and/or monosubstituted by $C_1$-$C_4$-alkoxy; $C_3$-$C_7$-alkynyl which may be monosubstituted to pentasubstituted by halogen and/or monosubstituted by $C_1$-$C_4$-alkoxy;

or a radical $NR^7R^8$, where

$R^7$ and $R^8$ are each hydrogen, $C_1$-$C_8$-alkyl, $C_1$-$C_8$-alkoxy, $C_1$-$C_6$-haloalkyl, $C_1$-$C_6$-haloalkoxy, aryl or hetaryl which may be monosubstituted to trisubstituted by halogen, $C_1$-$C_8$-alkyl, $C_1$-$C_8$-alkoxy, $C_1$-$C_6$-haloalkyl, $C_1$-$C_6$-haloalkoxy or alkoxycarbonylamino, or are each $C_1$-$C_{12}$-alkylcarbonyl, $C_1$-$C_{12}$-haloalkylcarbonyl, aroyl or hetaroyl which may be monosubstituted to trisubstituted by halogen, $C_1$-$C_5$-alkoxy, $C_1$-$C_4$-haloalkyl or $C_1$-$C_6$-alkyl,

or environmentally compatible salts thereof.

6. Use of a compound I as claimed in claim 5 as a herbicide.

## Revendications

### Revendications pour les Etats contractants suivants : CH, DE, FR, GB, IT, LI, NL

1. Dérivés du thiophène de formule générale I

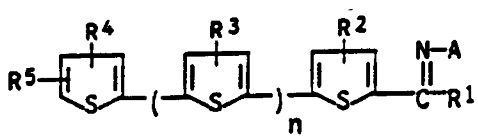

(I),

dans laquelle les symboles et l'indice ont les significations suivantes :

n est égal à 0 ou 1 ;

$R^1$ représente l'hydrogène, un atome d'halogène, un groupe alkyle en $C_1$-$C_8$, alcoxy en $C_1$-$C_6$, halogénoalkyle en $C_1$-$C_8$ ou halogénoalcoxy en $C_1$-$C_6$;

$R^2$, $R^3$, $R^4$ et $R^5$ représentent des groupes cyano, nitro ou les groupes mentionnés en référence à $R^1$ ;

A représente l'hydrogène, un groupe alkyle en $C_1$-$C_8$; halogénoalkyle en $C_1$-$C_6$, halogénoalkyle en $C_1$-$C_6$, alcoxy en $C_1$-$C_8$, halogénoalcoxy en $C_1$-$C_6$, un groupe aryle ou un groupe hétéroaryle dans lesquels les groupes aromatiques peuvent porter 1 à 5 atomes d'halogènes et/ou 1 à 3 des groupes suivants : alkyle en $C_1$-$C_8$, halogénoalkyle en $C_1$-$C_6$, alcoxy en $C_1$-$C_8$, halogénoalcoxy en $C_1$-$C_6$, hydroxycarbonylamino et/ou (alcoxy en $C_1$-$C_4$)-carbonylamino ;

un groupe $OR^6$ dans lequel

$R^6$ représente l'hydrogène, un groupe alkyle en $C_1$-$C_8$, un groupe alkyle en $C_1$-$C_4$ portant 1 à 5 substituants halogéno et/ou 1 substituant choisi parmi les groupes suivants : cyano, cycloalkyle en $C_3$-$C_7$, hydroxy, alcoxy en $C_1$-$C_4$, (alkyle en $C_1$-$C_4$)-carbonyl-alcoxy en $C_1$-$C_4$, (cycloalkyle en $C_3$-$C_7$)-carbonyl-alcoxy en $C_1$-$C_4$, (alkyle en $C_1$-$C_8$)-carbonyle, (halogénoalkyle en $C_1$-$C_8$)-carbonyle, (cycloalkyle en $C_3$-$C_8$)-carbonyle, (alcoxy en $C_1$-$C_8$)-carbonyle, mercapto, alkylthio en $C_1$-$C_4$, amino, alkylamino en $C_1$-$C_6$, di-(alkyle en $C_1$-$C_6$)-amino, ces groupes alkyle pouvant également former ensemble et avec l'atome d'azote un cycle aliphatique, aminoalcoxy en $C_1$-$C_4$, (alkyle en $C_1$-$C_4$)-amino-alcoxy en $C_1$-$C_4$, di-(alkyle en $C_1$-$C_4$)-amino-alcoxy en $C_1$-$C_4$, les groupes alkyle pouvant également former ensemble et avec l'atome d'azote un cycle aliphatique, ou un groupe hétéroaryle à 5 ou 6 chaînons contenant de 1 à 3 des hétéroatomes azote, oxygène et/ou soufre, ce cycle pouvant lui-même porter 1 à 5 substituants halogéno et/ou 1 à 3 des groupes suivants : alkyle en $C_1$-$C_4$, alcoxy en $C_1$-$C_4$, halogénoalkyle en $C_1$-$C_4$ et/ou halogénoalcoxy en $C_1$-$C_4$ ; cycloalkyle en $C_3$-$C_9$ ; alcényle en $C_2$-$C_8$ portant éventuellement 1 à 5 substituants halogéno et/ou 1 substituant alcoxy en $C_1$-$C_4$, alcynyle en $C_3$-$C_7$ éventuellement substitué par 1 à 5 atomes d'halogènes et/ou un groupe alcoxy en $C_1$-$C_4$ ;

ou bien un groupe $NR^7R^8$ dans lequel

$R^7$, $R^8$ représentent l'hydrogène des groupes alkyle en $C_1$-$C_8$, alcoxy en $C_1$-$C_8$, halogénoalkyle en $C_1$-$C_6$, halogénoalcoxy en $C_1$-$C_6$, aryle ou hétéroaryle qui peuvent porter 1 à 3 substituants halogéno, alkyle en $C_1$-$C_8$, alcoxy en $C_1$-$C_8$, halogénoalkyle en $C_1$-$C_6$, halogénoalcoxy en $C_1$-$C_6$ ou alcoxycarbonylamino, des groupes (alkyle en $C_1$-$C_{12}$)-carbonyle, (halogénoalkyle en $C_1$-$C_{12}$)-carbonyle, aroyle ou hétéroaroyle qui peuvent porter 1 à 3 substituants halogéno, alcoxy en $C_1$-$C_5$, halogénoalkyle en $C_1$-$C_4$ ou alkyle en $C_1$-$C_6$,

et leurs sels non polluants ;

sous réserve que, lorsque n est égal à 0,

a) A ne peut représenter un groupe aryle ou hétéroaryle éventuellement substitué lorsque $R^4$ représente l'hydrogène et que, simultanément, $R^5$ représente l'hydrogène, un groupe méthyle ou nitro, et

b) $R^7$ ou $R^8$ ne peut représenter un groupe phényle ou phényle substitué et $R^7$ et $R^8$ ne peuvent représenter tous deux des groupes phényle lorsque $R^1$, $R^2$, $R^4$ et $R^5$ représentent l'hydrogène.

2. Dérivés du thiophène de formule I de la revendication 1, dans laquelle n est égal à 0.

3. Procédé de préparation des composés de formule I selon revendication 1 ou 2, caractérisé en ce que l'on fait réagir un composé correspondant II

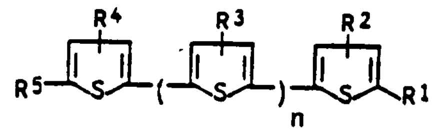

II,

de manière connue en soi avec un dérivé correspondant d'hydroxylamine ou d'hydrazine ou avec une amine primaire.

4. Produits herbicides contenant un dérivé du thiophène de formule I des revendications 1 et 2 et des additifs inertes.

5. Produits herbicides selon la revendication 4, contenant un dérivé du thiophène de formule I et d'autres constituants actifs.

6. Procédé pour combattre les croissances de végétaux adventices, caractérisé en ce que l'on traite les végétaux indésirables et/ou leur biotope par une quantité herbicide efficace d'un dérivé du thiophène de formule I

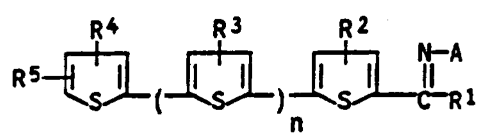

(I),

dans laquelle les symboles et l'indice ont les significations suivantes :

n est égal à 0 ou 1 ;

$R^1$ représente l'hydrogène, un atome d'halogène, un groupe alkyle en $C_1$-$C_8$, un groupe alcoxy en $C_1$-$C_6$, un groupe halogénoalkyle en $C_1$-$C_8$ ou halogénoalcoxy en $C_1$-$C_6$ ;

$R^2$, $R^3$, $R^4$ et $R^5$ représentent des groupes cyano, nitro ou les groupes mentionnés en référence à $R^1$;

A représente l'hydrogène, un groupe alkyle en $C_1$-$C_8$, halogénoalkyle en $C_1$-$C_6$, alcoxy en $C_1$-$C_8$, halogénoalcoxy en $C_1$-$C_6$, un groupe aryle ou un groupe hétéroaryle, les groupes aromatiques pouvant porter 1 à 5 atomes d'halogène et/ou 1 à 3 des groupes suivants : alkyle en $C_1$-$C_8$, halogénoalkyle en $C_1$-$C_6$, alcoxy en $C_1$-$C_8$, halogénoalcoxy en $C_1$-$C_6$, hydroxycarbonylamino et/ou (alcoxy en $C_1$-$C_4$)-carbonylamino ;

un groupe $OR^6$ dans lequel

$R^6$ représente l'hydrogène, un groupe alkyle en $C_1$-$C_8$, un groupe alkyle en $C_1$-$C_4$ substitué par 1 à 5 atomes d'halogènes et/ou par 1 des groupes suivants : cyano, cycloalkyle en $C_3$-$C_7$, hydroxy, alcoxy en $C_1$-$C_4$, (alkyle en $C_1$-$C_4$)-carbonylalcoxy en $C_1$-$C_4$, (cycloalkyle en $C_3$-$C_7$)-carbonylalcoxy en $C_1$-$C_4$, (alkyle en $C_1$-$C_8$)-carbonyle, (halogénoalkyle en $C_1$-$C_8$)-carbonyle, (cycloalkyle en $C_3$-$C_8$)-carbonyle, (alcoxy en $C_1$-$C_8$)-carbonyle, mercapto, alkylthio en $C_1$-$C_4$, amino, alkylamino en $C_1$-$C_6$, di-(alkyle en $C_1$-$C_6$)-amino, ces groupes alkyle pouvant également former ensemble et avec l'atome d'azote un cycle aliphatique, un groupe amino-alcoxy en $C_1$-$C_4$, (alkyle en $C_1$-$C_4$)-amino-alcoxy en $C_1$-$C_4$, di-(alkyle en $C_1$-$C_4$)-amino-alcoxy en $C_1$-$C_4$, les groupes alkyle pouvant également former ensemble et avec l'atome d'azote un cycle aliphatique, ou un groupe hétéroaryle à 5 ou 6 chaînons contenant 1 à 3 des hétéroatomes azote, oxygène et/ou soufre, ce cycle pouvant lui-même porter 1 à 5 substituants halogéno et/ou 1 à 3 substituants choisis parmi les groupes suivants : alkyle

en $C_1$-$C_4$, alcoxy en $C_1$-$C_4$, halogénoalkyle en $C_1$-$C_4$ et/ou halogénoalcoxy en $C_1$-$C_4$ ; cycloalkyle en $C_3$-$C_9$ ; alcényle en $C_2$-$C_8$ qui peut porter 1 à 5 substituants halogéno et/ou 1 substituant alcoxy en $C_1$-$C_4$ ; alcynyle en $C_3$-$C_7$ qui peut porter 1 à 5 substituants halogéno et/ou 1 substituant alcoxy en $C_1$-$C_4$ ;

ou bien un groupe $NR^7R^8$ dans lequel

$R^7$, $R^8$ représentent l'hydrogène, des groupes alkyle en $C_1$-$C_8$, alcoxy en $C_1$-$C_8$, halogénoalkyle en $C_1$-$C_6$, halogénoalcoxy en $C_1$-$C_6$, aryle ou hétéroaryle qui peuvent porter 1 à 3 substituants halogéno, alkyle en $C_1$-$C_8$, alcoxy en $C_1$-$C_8$, halogénoalkyle en $C_1$-$C_6$, halogénoalcoxy en $C_1$-$C_6$ ou alcoxycarbonylamino, des groupes (alkyle en $C_1$-$C_{12}$)-carbonyle, (halogénoalkyle en $C_1$-$C_{12}$)- carbonyle, aroyle ou hétéroaroyle qui peuvent porter 1 à 3 substituants halogéno, alcoxy en $C_1$-$C_5$, halogénoalkyle en $C_1$-$C_4$ ou alkyle en $C_1$-$C_6$,

ou de leurs sels non polluants.

7. Utilisation d'un composé I de la revendication 6 en tant qu'herbicide.

**Revendications pour l'Etat contractant suivant : ES**

1. Procédé de préparation de dérivés du thiophène de formule générale I

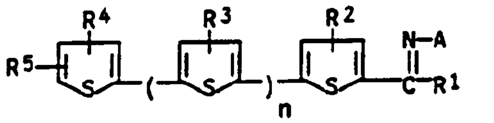

(I),

dans laquelle les symboles et l'indice ont les significations suivantes :

n est égal à 0 ou 1 ;

$R^1$ représente l'hydrogène, un atome d'halogène, un groupe alkyle en $C_1$-$C_8$, alcoxy en $C_1$-$C_6$, halogénoalkyle en $C_1$-$C_8$ ou halogénoalcoxy en $C_1$-$C_6$ ;

$R^2$, $R^3$, $R^4$ et $R^5$ représentent des groupes cyano, nitro ou les groupes mentionnés en référence à $R^1$ ;

A représente l'hydrogène, un group alkyle en $C_1$-$C_8$, halogénoalkyle en $C_1$-$C_6$, alcoxy en $C_1$-$C_8$, halogénoalcoxy en $C_1$-$C_6$, un groupe aryle ou un groupe hétéroaryle, les groupes aromatiques pouvant porter 1 à 5 atomes d'halogènes et/ou 1 à 3 des groupes suivants : alkyle en $C_1$-$C_8$, halogénoalkyle en $C_1$-$C_6$, alcoxy en $C_1$-$C_8$, halogénoalcoxy en $C_1$-$C_6$, hydroxycarbonylamino et/ou (alcoxy en $C_1$-$C_4$)-carbonylamino ;

un groupe $OR^6$ dans lequel

$R^6$ représente l'hydrogène, un groupe alkyle en $C_1$-$C_8$, un groupe alkyle en $C_1$-$C_7$ qui peut porter 1 à 5 substituants halogéno et/ou 1 des substituants suivants : cyano, cycloalkyle en $C_3$-$C_7$, hydroxy, alcoxy en $C_1$-$C_4$, (alkyle en $C_1$-$C_4$)-carbonylalcoxy en $C_1$-$C_4$, (cycloalkyle en $C_3$-$C_7$)-carbonylalcoxy en $C_1$-$C_4$, (alkyle en $C_1$-$C_8$)-carbonyle, (halogénoalkyle en $C_1$-$C_8$)-carbonyle, (cycloalkyle en $C_3$-$C_8$)-carbonyle, (alcoxy en $C_1$-$C_8$)-carbonyle, mercapto, alkythio en $C_1$-$C_4$, amino, alkylamino en $C_1$-$C_6$, di-(alkyle en $C_1$-$C_6$)-amino, ces groupes alkyle pouvant également former ensemble et avc l'atome d'azote un cycle aliphatique, un groupe aminoalcoxy en $C_1$-$C_4$, (alkyle en $C_1$-$C_4$)-aminoalcoxy en $C_1$-$C_4$, di-(alkyle en $C_1$-$C_4$)-aminoalcoxy en $C_1$-$C_4$, les groupes alkyle pouvant également former ensemble et avec l'atome d'azote un cycle aliphatique, ou un groupe hétéroaryle à 5 ou 6 chaînons contenant 1 à 3 des hétéroatomes azote, oxygène et/ou soufre, ce cycle pouvant lui-même porter 1 à 5 substituants halogéno et/ou 1 à 3 des substituants suivants : alkyle en $C_1$-$C_4$, alcoxy en $C_1$-$C_4$, halogénoalkyle en $C_1$-$C_4$ et/ou halogénoalcoxy en $C_1$-$C_4$ ; cycloalkyle en $C_3$-$C_9$ ; alcényle en $C_2$-$C_8$ pouvant porter 1 à 5 substituants halogéno et/ou 1 substituant alcoxy en $C_1$-$C_4$ ; alcynyle en $C_3$-$C_7$ qui peut porter 1 à 5 substituants halogéno et/ou un substituant alcoxy en $C_1$-$C_4$ ;

ou bien un groupe $NR^7R^8$ dans lequel

$R^7$, $R^8$ représentent l'hydrogène, des groupes alkyle en $C_1$-$C_8$, alcoxy en $C_1$-$C_8$, halogénoalkyle en $C_1$-$C_6$, halogénoalcoxy en $C_1$-$C_6$, aryle ou hétéroaryle qui peuvent porter 1 à 3 substituants halogéno, alkyle en $C_1$-$C_8$ alcoxy en $C_1$-$C_8$, halogénoalkyle en $C_1$-$C_6$, halogénoalcoxy en $C_1$-$C_6$ ou alcoxycarbonylamino, des groupes (alkyle en $C_1$-$C_{12}$)-carbonyle, (halogénoalkyle en $C_1$-$C_{12}$)-carbonyle, aroyle ou hétéroaroyle qui peuvent porter 1 à 3 substituants halogéno, alcoxy en $C_1$-$C_5$, halogénoalkyle en $C_1$-$C_4$ ou alkyle en $C_1$-$C_6$,

et de leurs sels non polluants, sous réserve que,

lorsque n est égal à 0,

a) A ne peut représenter un groupe aryle ou hétéroaryle éventuellemen substitué lorsque $R^4$ représente l'hydrogène et que, simultanément, $R^5$ représente l'hydrogène, un groupe méthyle ou nitro, et

b) $R^7$ ou $R^8$ ne peut représenter un groupe phényle ou phényle substitué et $R^7$ et $R^8$ ne peuvent représenter tous deux des groupes phényle lorsque $R^1$, $R^2$, $R^4$ et $R^5$ représentent l'hydrogène, caractérisé en ce que l'on fait réagir un composé correspondant II

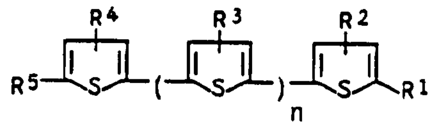

II,

de manière connue en soi, avec un dérivé correspondant de l'hydroxyalmine ou de l'hydrazine ou avec une amine primaire.

2. Procédé de préparation des dérivés du thiophène de formule I de la revendication 1 dans laquelle n est égal à 0.

3. Produits herbicides contenant un dérivé du thiophène de formule I selon revendications 1 et 2 et des additifs inertes.

4. Produits herbicides selon revendication 3, contenant un dérivé du thiophène de formule I et d'autres constituants actifs.

5. Procédé pour combattre les croissances de végétaux indésirables, caractérisé en ce que l'on traite les végétaux indésirables et/ou leur biotope par une quantité herbicide efficace d'un dérivé du thiophène de formule I

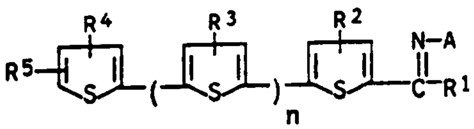

(I),

dans laquelle les symboles et l'indice ont les significations suivantes :

n est égal à 0 ou 1 ;

$R^1$ représente l'hydrogène, un atome d'halogène, un groupe alkyle en $C_1$-$C_8$, alcoxy en $C_1$-$C_6$, halogénoalkyle en $C_1$-$C_8$ ou halogénoalcoxy en $C_1$-$C_6$ ;

$R^2$, $R^3$, $R^4$ et $R^5$ représentent des groupes cyano, nitro ou les groupes mentionnés en référence à $R^1$;

A représente l'hydrogène, un groupe alkyle en $C_1$-$C_8$, halogénoalkyle en $C_1$-$C_6$, alcoxy en $C_1$-$C_8$, halogénoalcoxy en $C_1$-$C_6$, un groupe aryle ou hétéroaryle, les groupes aromatiques pouvant porter 1 à 5 atomes d'halogènes et/ou 1 à 3 des groupes suivants : alkyle en $C_1$-$C_8$, halogénoalkyle en $C_1$-$C_6$, alcoxy en $C_1$-$C_8$, halogénoalcoxy en $C_1$-$C_6$, hydroxycarbonylamino et/ou (alcoxy en $C_1$-$C_4$)-carbonylamino ;

un groupe $OR^6$ dans lequel

$R^6$ représente l'hydrogène, un groupe alkyle en $C_1$-$C_8$, un groupe alkyle en $C_1$-$C_4$ qui peut porter 1 à 5 substituants halogéno et/ou 1 substituant choisi parmi les groupes suivants : cyano, cycloalkyle en $C_3$-$C_7$, hydroxy-alcoxy en $C_1$-$C_4$, (alkyle en $C_1$-$C_4$)-carbonyl-alcoxy en $C_1$-$C_4$, (cycloalkyle en $C_3$-$C_7$)-carbonyl-alcoxy en $C_1$-$C_4$, (alkyle en $C_1$-$C_8$)-carbonyle, (halogénoalkyle en $C_1$-$C_8$)-carbonyle, (cycloalkyle en $C_3$-$C_8$)-carbonyle, (alcoxy en $C_1$-$C_8$)-carbonyle, mercapto, alkylthio en $C_1$-$C_4$, amino, alkylamino en $C_1$-$C_6$, di-(alkyle en $C_1$-$C_6$)-amino, ces groupes alkyle pouvant également former ensemble et avec l'atome d'azote un cycle aliphatique, aminoalcoxy en $C_1$-$C_4$, (alkyle en $C_1$-$C_4$)-amino-alcoxy en $C_1$-$C_4$, di-(alkyle en $C_1$-$C_4$)-amino-alcoxy en $C_1$-$C_4$, les groupes alkyle pouvant également former ensemble et avec l'atome d'azote un cycle aliphatique, ou un groupe hétéroaryle à 5 ou 6 chaînons contenant 1 à 3 des hétéroatomes azote, oxygène et/ou soufre, ce cycle pouvant lui-même porter 1 à 5 substituants halogéno et/ou 1 à 3 des substituants suivants : alkyle en $C_1$-$C_4$, alcoxy en $C_1$-$C_4$, halogénoalkyle en $C_1$-$C_4$ et/ou halogénoalcoxy en $C_1$-$C_4$ ; cycloalkyle en $C_3$-$C_9$ ; alcényle en $C_2$-$C_8$ pouvant porter 1 à 5 substituants halogéno et/ou un substituant alcoxy en $C_1$-$C_4$ ; un groupe alcynyle en $C_3$-$C_7$ pouvant porter 1 à 5 substituants halogéno et/ou un substituant alcoxy en $C_1$-$C_4$ ;

ou un groupe $NR^7R^8$ dans lequel

$R^7$, $R^8$ représentent l'hydrogène, des groupes alkyle en $C_1$-$C_8$, alcoxy en $C_1$-$C_8$, halogénoalkyle en $C_1$-$C_6$, halogénoalcoxy en $C_1$-$C_6$, aryle ou hétéroaryle qui peuvent porter 1 à 3 substituants halogéno, alkyle en $C_1$-$C_8$, alcoxy en $C_1$-$C_8$, halogénoalkyle en $C_1$-$C_6$, halogénoalcoxy en $C_1$-$C_6$ ou alcoxycarbonylamino des groupes (alkyle en $C_1$-$C_{12}$)-carbonyle, (halogénoalkyle en $C_1$-$C_{12}$)- carbonyle, aroyle ou hétéroaroyle qui peuvent porter 1 à 3 substituants halogéno alcoxy en $C_1$-$C_5$, halogénoalkyle en $C_1$-$C_4$ ou alkyle en $C_1$-$C_6$,

ou de leurs sels non polluants

6. Utilisation d'un composé I de la revendication 5 en tant qu'herbicide.